# EUROPEAN PATENT APPLICATION

(11) **EP 3 693 033 A1**
(43) Date of publication of application: **12.08.2020**
(21) Application number: 18864482.7
(22) Date of filing: 01.10.2018
(51) Int. Cl.: A61L 31/04, A61K 9/08, A61K 35/22, A61K 35/38, A61K 47/30, A61L 31/14, A61L 31/16, A61P 13/02

(54) **URETHRAL STENOSIS TREATMENT AGENT AND URETHRAL STENOSIS TREATMENT METHOD**

(30) Priority: 06.10.2017 JP 2017195713
(71) Applicant: GN Corporation Ltd, Kofu-shi, Yamanashi 400-0866 (JP); JBM Incorporation, Tokyo 133-0052 (JP)
(72) Inventor: YOSHIOKA Hiroshi, Hadano-shi Kanagawa 257-0026 (JP); Samuel Abraham JK, Kofu-shi Yamanashi 400-0866 (JP)
(74) Representative: Mewburn Ellis LLP
(86) International application number: PCT/JP2018/036709
(87) International publication number: WO 2019/069858

(57) **Abstract**

[Object]

The present invention is intended to provide a urethral stricture treatment agent and a urethral stricture treatment method capable of avoiding restenosis by a less invasive transurethral endoscopic procedure in urethral stricture treatment.

[Solution]

A urethral stricture treatment agent including at least a hydrogel-forming polymer, and having a storage elastic modulus of 50 Pa or less at 10°C and a storage elastic modulus of 100 Pa or more at 37°C.

A urethral stricture treatment method at least including injecting the urethral stricture treatment agent cooled to 10°C or lower into an inner surface of urethra which has been incised with a transurethral endoscopic procedure, and holding the urethral stricture treatment agent in the inner surface of urethra at a temperature not lower than a room temperature.

[Selected Drawing]

None

## Description

### TECHNICAL FIELD

The present invention relates to a treatment agent effective for treatment of urethral stricture. The present invention also relates to a method for treating urethral stricture.

### BACKGROUND ART

Urethral stricture is caused by various factors such as secondary damage due to surgery with a urethral endoscope on prostatic hypertrophy or bladder cancer, external wounds caused by traffic accidents or accidents during labor work, and hypospadias, which is a congenital urethral disease. In urethral stricture, the urethral mucosa is damaged by an injury or inflammation, and the urethral mucosa and the urethral corpus cavernosum surrounding the urethral mucosa are scarred in the course of cure of the damage, resulting in narrowing of the urethra.

Examples of the method for treating urethral stricture include surgical reconstruction of urethra, but this method is highly invasive and requires long-term hospitalization. Therefore, recently, a less invasive and transurethral endoscopic dilation procedure using, for example, a simple bougie (urethral dilator), a balloon catheter, a cold knife, or a laser has been performed (Internal Urethrotomy for Strictures of the Male Urethra, Shigeaki Hayashida, Tadao Kiriyama, Hiroshi Hironaka, Shizuka Kikkawa, Acta Urologica Japonica (1972), 18(8): 588-593).

### CITATION LIST

### NON-PATENT LITERATURE

Non-Patent Literature 1: Internal Urethrotomy for Strictures of the Male Urethra, Shigeaki Hayashida, Tadao Kiriyama, Hiroshi Hironaka, Shizuka Kikkawa, Acta Urologica Japonica (1972), 18(8): 588-593

### SUMMARY OF INVENTION

### TECHNICAL PROBLEMS

However, the above-described conventional treatment method has a problem of recurrence of urethral stricture because the capacity to reconstruct epithelium cells on the inner surface of the scarred urethra is extremely low. An object of the present invention is to provide a urethral stricture treatment agent and a urethral stricture treatment method which can avoid restenosis using a less invasive transurethral endoscopic procedure in treatment of urethral stricture.

### SOLUTIONS TO PROBLEMS

The inventors have found that, as a method for treating urethral stricture, retention of hydrogel having specific properties in the inner surface of urethra, which has been incised by a transurethral endoscopic procedure, promotes epithelization of the incised site, and effectively prevents recurrence of urethral stricture that would otherwise be caused by scarring of the site, and thus completed the present invention.

Additionally, the inventors have found that inclusion of animal cells in the hydrogel is effective for solving the above-described problems. They have found that it is particularly effective when the animal cells are oral mucosal cells of the patient himself/herself.

More specifically, the problem of the present invention is solved by a urethral stricture treatment agent including at least a hydrogel-forming polymer, the agent having a storage elastic modulus of 50 Pa or less at 10°C, and a storage elastic modulus of 100 Pa or more at 37°C.

Furthermore, the problem of the present invention is solved by the above-described urethral stricture treatment agent including animal cells.

The problem of the present invention is solved also by a urethral stricture treatment agent in which the animal cells are oral mucosal cells of the patient.

The problem of the present invention is solved also by a urethral stricture treatment method including injecting a urethral stricture treatment agent cooled to 10°C or lower, which includes at least a hydrogel-forming polymer and has a storage elastic modulus of 50 Pa or less at 10°C and a storage elastic modulus of 100 Pa or more at 37°C, into the inner surface of urethra which has been incised with a transurethral endoscopic procedure, and holding the agent in the inner surface of urethra at a temperature not lower than the room temperature.

### ADVANTAGEOUS EFFECTS OF INVENTION

As described above, according to the present invention, retention of hydrogel having specific properties in the inner surface of urethra, which has been incised by a transurethral endoscopic procedure, promotes epithelization of the incised site, and effectively prevents recurrence of urethral stricture that would otherwise be caused by scarring of the site.

Furthermore, epithelization of the incised site in the inner surface of urethra is further promoted by inclusion of animal cells (particularly oral mucosal cells of the patient) in the hydrogel.

### DESCRIPTION OF EMBODIMENTS

The present invention will be described below in detail.

### (Hydrogel-forming polymer)

The "hydrogel-forming polymer" of the present invention refers to a polymer that thermally reversibly forms a crosslinking structure or a network structure, and can thermally reversibly form hydrogel retaining a dispersed liquid such as water within the polymer based on the structure. The "hydrogel" refers to a gel including a crosslinking or network structure made of a polymer and water supported or held in the structure.

### (Storage elastic modulus)

In the present invention, measurement of the storage elastic modulus of hydrogel can be achieved by the method described in a literature (H. Yoshioka et al., Journal of Macromolecular Science, A31 (1), 113 (1994)). More specifically, the dynamic modulus of elasticity of a sample at an observation frequency of 1 Hz is measured at a predetermined temperature (10°C, 25°C, or 37°C), and the storage elastic modulus of the sample (G', elastic term) is determined. In this measurement, the following measurement conditions are preferred.

### <Measurement conditions of dynamic loss elastic modulus>

Measurement instrument (trade name): stress controlled rheometer AR500, manufactured by TA Instruments)
Amount of sample solution: about 0.8 g
Shape and dimension of cell for measurement: acrylic parallel disk (diameter: 4.0 cm), gap: 600 µm
Measurement frequency: 1 Hz
Application stress: within linear site.

The urethral stricture treatment agent of the present invention has a storage elastic modulus of 50 Pa or less, preferably 30 Pa or less (particularly preferably 10 Pa or less) at 10°C, and a storage elastic modulus of 100 Pa or more, preferably 200 Pa or more (particularly preferably 300 Pa or more) at 37°C.

The urethral stricture treatment agent of the present invention is injected into the site with urethral stricture to be treated at a low temperature of 10°C or lower, and the urethral stricture treatment agent is held in the site with urethral stricture to be treated at body temperature. If the storage elastic modulus of the urethral stricture treatment agent at 10°C is more than 50 Pa, the hardness of the agent is too high, which makes it difficult to inject the agent through a catheter.

On the other hand, if the storage elastic modulus of the urethral stricture treatment agent of the present invention at 37°C is less than 100 Pa, the strength of the agent is insufficient, which makes it difficult to hold the agent for a long period of time in the site with urethral stricture to be treated.

Furthermore, the site having urethral stricture to be treated in male patients is often located in the penis, and thus is exposed to outside the body and susceptible to the influence of outside air temperature. If the storage elastic modulus of the aqueous solution of the "hydrogel-forming polymer" of the present invention at room temperature (25°C) is below 100 Pa, the hydrogel is readily fluidized by the decrease of the outside air temperature, so that retention of animal cells in the site having urethral stricture to be treated becomes imperfect. As a result, the scarred inner surface of urethra has insufficient capability of reconstructing epithelial cells, which can cause the problem of recurrence of urethral stricture. Accordingly, the storage elastic modulus of the urethral stricture treatment agent of the present invention is preferably 100 Pa or more, and preferably 200 Pa or more (particularly preferably 300 Pa or more) at 25°C.

"The hydrogel-forming polymer" that imparts a favorable storage elastic modulus described above to the urethral stricture treatment agent of the present invention can be easily selected from the specific compounds described below according to the above-described screening method (storage elastic modulus measurement method).

Specific examples of known polymers whose hydrogel reversibly exhibits flowability at a lower temperature include polyalkylene oxide block copolymers such as a block copolymer of polypropylene oxide and polyethylene oxide; etherified cellulose such as methyl cellulose and hydroxypropyl cellulose; and chitosan derivatives (K, R, Holme, et al. Macromolecules, 24, 3828 (1991)).

### (Favorable hydrogel-forming polymer)

A hydrogel-forming polymer using hydrophobic bonding for crosslinking, which is suitable as the "hydrogel-forming polymer" of the present invention, is preferably composed of multiple blocks having a cloud point and a hydrophilic block.

The presence of the hydrophilic block is preferred so as to make the hydrogel water-soluble at a lower temperature, and the presence of the multiple blocks having a cloud point is preferred so as to cause gelation of the hydrogel at a higher temperature. In other words, the blocks having a cloud point are soluble in water at temperatures lower than the cloud point, and become insoluble in water at temperatures higher than the cloud point, so that the blocks work as crosslinking points including hydrophobic bonds for forming a gel at temperatures higher than the cloud point.

The hydrogel used in the present invention utilizes the properties that the hydrophobic bonds become stronger with an increase in the temperature, and that the change is reversible depending on the temperature. The "hydrogel-forming polymer" preferably has multiple "blocks having a cloud point", thereby forming multiple crosslinking points in one molecule, and forming a highly stable gel.

Meanwhile, the hydrophilic block in the "hydrogel-forming polymer" has, as described above, a function of making the "hydrogel-forming polymer" water-soluble at a lower temperature, and also has a function of forming the state of a hydrous gel while preventing flocculation and precipitation of the hydrogel that would otherwise be caused by too much increase of the hydrophobic bonding strength at a temperature higher than the transition temperature.

The "hydrogel-forming polymer" used in the present invention is preferably decomposed and absorbed in vivo. More specifically, it is preferred that the "hydrogel-forming polymer" of the present invention be decomposed by hydrolysis reaction or enzyme reaction in vivo, and absorbed and excreted in the form of a low molecular weight molecule that is biologically harmless.

When the "hydrogel-forming polymer" of the present invention includes multiple blocks having a cloud point bonded to a hydrophilic block, it is preferred that at least either the blocks having a cloud point or the hydrophilic block, preferably both, be decomposed and absorbed in vivo.

### (Multiple blocks having a cloud point)

The blocks having a cloud point are preferably polymeric blocks having a negative solubility-temperature coefficient to water. More specifically, preferred are polymers selected from the group consisting of copolymers of polypropylene oxide or propylene oxide and other alkylene oxide, copolymers of poly-N-substituted acrylamide derivatives, poly-N-substituted methacrylamide derivatives, N-substituted acrylamide derivatives and N-substituted methacrylamide derivatives, polyvinyl methyl ether, partially acetylated polyvinyl alcohols.

In order to make the blocks having a cloud point decomposable and absorbable in vivo, it is effective if the blocks having a cloud point are polypeptides including a hydrophobic amino acid and a hydrophilic amino acid. Alternatively, a polyester-type biodegradable polymer such as polylactic acid or polyglycolic acid may be used as the blocks having a cloud point decomposed and absorbed in vivo.

The cloud point of the polymer (blocks having a cloud point) is preferably higher than 4°C and 40°C or less, from the viewpoint of making the storage elastic modulus of the polymer used in the present invention (a compound including multiple blocks having a cloud point bonded to a hydrophilic block) a desired value at a predetermined temperature.

Measurement of the cloud point can be achieved by, for example, cooling an aqueous solution of about 1% by mass of the polymer (blocks having a cloud point) to make a transparent uniform solution, and then gradually increasing the temperature of the solution (temperature rising rate: about 1°C/min), and recording the point when the solution is turned cloudy first as the cloud point.

Specific examples of the poly-N-substituted acrylamide derivative and poly-N-substituted methacrylamide derivative that can be used in the present invention are listed below.

Poly-N-acroyl piperidine; poly-N-n-propyl methacrylamide; poly-N-isopropyl acrylamide; poly-N,N-diethylacrylamide; poly-N-isopropylmethacrylamide; poly-N-cyclopropylacrylamide; poly-N-acryloylpyrrolidine; poly-N,N-ethylmethylacrylamide; poly-N-cyclopropylmethacrylamide; and poly-N-ethylacrylamide.

These polymers may be a homopolymer or a copolymer of a monomer composing the above-described polymer and other monomer. Other monomer composing the copolymer may be a hydrophilic monomer or a hydrophobic monomer. In general, copolymerization with a hydrophilic monomer increases the cloud point of the product, and copolymerization with a hydrophobic monomer decreases the cloud point of the product. Accordingly, a polymer having a desired cloud point (for example, a cloud point higher than 4°C and 40°C or lower) can be obtained by appropriately selecting the monomer to be copolymerized.

### (Hydrophilic monomer)

Examples of the hydrophilic monomer include, but are not limited to, N-vinylpyrrolidone, vinylpyridine, acrylamide, methacrylamide, N-methylacrylamide, hydroxyethyl methacrylate, hydroxyethyl acrylate, hydroxymethyl methacrylate, hydroxymethyl acrylate; acrylic acid, methacrylic acid and salts thereof, vinylsulfonic acid, and styrenesulfonic acid having an acidic group; N,N-dimethylaminoethyl methacrylate, N,N-diethylaminoethyl methacrylate, N,N-dimethylaminopropyl acrylamide, and salts thereof having a basic group.

### (Hydrophobic monomer)

Examples of the hydrophobic monomer include, but are not limited to, acrylate derivatives and methacrylate derivatives such as ethyl acrylate, methyl methacrylate, and glycidyl methacrylate; N-substituted alkyl methacrylamide derivatives such as N-n-butyl methacrylamide; vinyl chloride, acrylonitrile, styrene, and vinyl acetate.

### (Hydrophilic block)

Meanwhile, specific examples of the above-described hydrophilic block to be bonded to the blocks having a cloud point include methyl cellulose, dextran, polyethylene oxide, polyvinyl alcohol, poly-N-vinylpyrrolidone, polyvinyl pyridine, polyacrylamide, polymethacrylamide, poly-N-methylacrylamide, polyhydroxymethyl acrylate, polyacrylic acid, polymethacryl acid, polyvinylsulfonic acid, polystyrenesulfonic acid, and salts thereof; poly-N,N-dimethylaminoethyl methacrylate, poly-N,N-diethylaminoethyl methacrylate, poly-N,N-dimethylaminopropylacryl amide, and salts thereof.

The hydrophilic block is preferably decomposed, metabolized, and excreted in vivo; preferred ones are hydrophilic biopolymers such as proteins such as albumin and gelatin, and polysaccharides such as hyaluronic acid, heparin, chitin, and chitosan.

The method for bonding the blocks having a cloud point and the hydrophilic block is not particularly limited. The bonding can be achieved by, for example, introducing a polymerizable functional group (for example, an acryloyl group) to either of the blocks described above, and copolymerizing the block with a monomer giving the other block. The bonded product of the blocks having a cloud point and the hydrophilic block can be obtained by block copolymerization of a monomer giving the blocks having a cloud point and a monomer giving the hydrophilic block. Alternatively, bonding between the blocks having a cloud point and the hydrophilic block can be achieved by introducing a reactive functional group (for example, a hydroxyl group, an amino group, a carboxyl group, or an isocyanate group) to both blocks, and bonding them by chemical reaction. At this time, usually, multiple reactive functional groups are introduced to the hydrophilic block. Bonding between polypropylene oxide having a cloud point and the hydrophilic block can be achieved by, for example, repeated sequential polymerization of propylene oxide and a monomer composing "other hydrophilic block" (for example, ethylene oxide) by anionic polymerization or cationic polymerization, thereby obtaining a block copolymer in which polypropylene oxide and "hydrophilic block" (for example, polyethylene oxide) are bonded. These block copolymers can be obtained also by introducing a polymerizable group (for example, an acryloyl group) to the terminal of polypropylene oxide, and then copolymerizing the monomer composing the hydrophilic block. Furthermore, the polymer used in the present invention can be obtained by introducing a functional group, which can cause bonding reaction with the functional group (for example, hydroxyl group) at the terminal of polypropylene oxide, to the hydrophilic block, and allowing them to react. The "hydrogel-forming polymer" used in the present invention can be obtained by bonding materials such as PLURONIC F-127 (trade name, manufactured by Asahi Denka Co., Ltd.) in which polyethylene glycol is bonded to both ends of polypropylene glycol.

In the polymer of the present invention according to an aspect including the blocks having a cloud point, the "blocks having a cloud point" existing in the molecule are water-soluble together with the hydrophilic block at a temperature lower than the cloud point, and thus completely dissolve in water and exhibit a sol state. However, if the temperature of the aqueous solution of the polymer is increased to a temperature higher than the above-described cloud point, the "blocks having a cloud point" in the molecule turn hydrophobic, and associate with different molecules due to hydrophobic interaction.

Meanwhile, the hydrophilic block is water-soluble at this point (the point when heated to a temperature higher than the cloud point), the polymer of the present invention forms hydrogel in water, the hydrogel having a three-dimensional network structure crosslinked at the hydrophobic association points between the blocks having a cloud point. When the temperature of the hydrogel is decreased again to a temperature lower than the cloud point of the "blocks having a cloud point" existing in the molecule, the blocks having a cloud point turn water-soluble, the crosslinking points by hydrophobic association are released, the hydrogel structure disappears, and the "hydrogel-forming polymer" of the present invention becomes again a complete aqueous solution. In this manner, physical property change in the polymer of the present invention in a preferred aspect is based on the reversible change between hydrophilicity and hydrophobicity at the cloud point of the blocks having a cloud point existing in the molecule, and thus has complete reversibility according to the temperature change.

According to the study by the inventors, the delicate balance between hydrophilicity and hydrophobicity of the "hydrogel-forming polymer" in water seems to contribute to stability of cells during cultivation of the cells.

### (Solubility of gel)

As described above, the hydrogel-forming polymer of the present invention exhibits substantial water insolubility at body temperature (37°C), and exhibits reversible water solubility under cooling with ice. Regarding the above-described "substantial water insolubility", the amount of the polymer dissolved in 100 mL of water at 37°C is preferably 5.0 g or less (more preferably 0.5 g or less, and particularly preferably 0.1 g or less).

On the other hand, regarding the above-described "water solubility" under cooling with ice, the amount of the polymer dissolved in 100 mL of water at 10°C is preferably 0.5 g or more (more preferably 1.0 g or more).

The "reversible water solubility" means that the aqueous solution of the "hydrogel-forming polymer" exhibits the above-described water solubility at 10°C even after once turning into a "substantially water-insoluble" gel at 37°C.

The 10% aqueous solution of the polymer preferably has a viscosity of 10 to 3,000 cP (more preferably 50 to 1.000 cP) at 5°C. The viscosity is preferably measured under, for example, the following measurement conditions.
Viscometer: stress controlled rheometer (model name: AR500, manufactured by TA Instruments)
Rotor diameter: 60 mm
Rotor shape: parallel plate

When the aqueous solution of the "hydrogel-forming polymer" of the present invention is immersed in a plenty of water at 37°C, the gel will not be substantially dissolved. The above-described properties of the hydrogel formed by the "hydrogel-forming polymer" can be confirmed by, for example, as follows. More specifically, 0.15 g of the "hydrogel-forming polymer" is dissolved in 1.35 g of distilled water under cooling with ice, thereby making a 10 wt% aqueous solution. The aqueous solution is injected into a plastic petri dish having a diameter of 35 mm, and humidified at 37°C, thereby making a gel having a thickness of about 1.5 mm in the petri dish. Thereafter, the weight of the whole petri dish containing the gel (f gram) is measured. Subsequently, the whole petri dish containing the gel is allowed to stand in 250 mL of water at 37°C for 10 hours, and then the weight of the whole petri dish containing the gel (g gram) is measured, thereby evaluating the presence or absence of dissolution of the gel from the gel surface. At this time, in the hydrogel-forming polymer of the present invention, the weight decrement of the gel, or (f - g)/f is preferably 5.0% or less, and more preferably 1.0% or less (particularly preferably 0.1% or less).

After the aqueous solution of the "hydrogel-forming polymer" of the present invention is gelated at 37°C, the gel will not dissolve over a long period if it is immersed in a plenty amount of water (about 0.1 to 100 times the gel in terms of the volume ratio). Such properties of the polymer used in the present invention are achieved by, for example, the presence of two or more (multiple) blocks having a cloud point in the polymer.

On the other hand, the inventors have found that a similar gel made using the above-described PLURONIC F-127 composed of polypropylene oxide having polyethylene oxide bonded to both ends completely dissolves in water after standing for several hours.

From the viewpoint of minimizing cytotoxicity in a non-gel state, it is preferable to use a "hydrogel-forming polymer" which can be gelated at a concentration of 20% or less (more preferably 15% or less, particularly preferably 10% or less) in terms of the concentration in water, or {(polymer)/(polymer + water)} × 100 (%).

The molecular weight of the "hydrogel-forming polymer" used in the present invention is preferably 30,000 or more 30,000,000 or less, more preferably 100,000 or more and 10,000,000 or less, and even more preferably 500,000 or more and 5,000,000 or less.

Adjustment of the storage elastic modulus of the urethral stricture treatment agent of the present invention to a preferable range can be achieved by, as described above, selecting the kind of "hydrogel-forming polymer", and adjusting the concentration of the "hydrogel-forming polymer" in the urethral stricture treatment agent. Usually, the storage elastic modulus of the urethral stricture treatment agent of the present invention is increased by increasing the concentration of the "hydrogel-forming polymer", and decreased by decreasing the concentration.

### (Additive salt)

The urethral stricture treatment agent of the present invention includes at least the above-described "hydrogel-forming polymer", and preferably further includes salts such as a pH buffer and a normal saline solution, in order to have a pH and an osmotic pressure close to those of a biogenic body fluid.

### (Animal cells)

The urethral stricture treatment agent of the present invention may include dispersed animal cells. The animal cells are most preferably urethral mucosal epithelial cells. In order to facilitate collection and avoid immune rejection reaction, the use of self oral mucosal cells is preferred.

In addition to differentiated cells, undifferentiated cells may be used because they have high immune tolerance as animal cells. Examples of the undifferentiated cells include pluripotency stem cells such as ES cells and iPS cells, and mesenchymal stem cells.

In the urethral stricture treatment agent of the present invention, animal cells may be added immediately before use as the urethral stricture treatment agent. Alternatively, the cells may be applied to the urethral stricture site after being preliminarily dispersed in the urethral stricture treatment agent and proliferated.

When the cells are undifferentiated cells, they may be proliferated in the state of being undifferentiated, and then induced to differentiation to the lineage of mucosal epithelial cells.

### (Cytokine)

The urethral stricture treatment agent of the present invention may contain any cytokine for the purpose of promoting mucosal cell epithelialization of the urethral stricture site. Since cytokine is water-soluble, it is readily dispersed if injected as it is into the urethral stricture site. However, the urethral stricture treatment agent of the present invention has a more closely packed polymer network formed by the "hydrogel-forming polymer", whereby diffusion of cytokine is suppressed. Therefore, cytokine is held around the urethral stricture site at a high concentration, whereby the effect of cytokine is maintained for a long period of time.

The cytokine preferred in the present invention is not particularly limited as long as it promotes mucosal cell epithelialization of the urethral stricture site, and is preferably those having actions such as maintenance of undifferentiation of cells, promotion of proliferation, and promotion of differentiation induction to the lineage of urethral mucosal epithelial cells.

### (Urethral stricture treatment method)

The urethral stricture treatment agent of the present invention is held in the inner surface of urethra which has been incised with a transurethral endoscopic procedure, thereby promoting epithelialization of the incised site and effectively preventing recurrence of urethral stricture that would otherwise be caused by turning the site to scar tissues.

The urethral stricture site of the patient was incised by an ordinary transurethral endoscopic incision procedure, a urethra catheter was inserted, and then the urethral stricture treatment agent of the present invention cooled with ice is injected between the periphery of the urethra catheter and the incised site of the inner surface of urethra through a different catheter from the urethra catheter. The urethral stricture treatment agent of the present invention is warmed by body temperature and instantly gelated, and held so as to cover the whole incised site of the inner surface of urethra. When the urethra catheter is extracted three weeks after surgery, the site with incised stricture is covered by mucosal epithelial cells without causing scarring, and good flow of urine is ensured.

The urethral stricture treatment agent of the present invention is soluble in ice-cold water. Therefore, when the urethral stricture treatment agent of the present invention is to be removed for some reason, it can be easily washed away with ice-cold water.

### EXAMPLES

The present invention will be further described below with reference to preparation examples of the "hydrogel-forming polymer" and examples of the present invention, but the scope of the invention is only limited by CLAIMS, and will not be limited by the following examples.

### Preparation Example 1

10 g of a polypropylene oxide-polyethylene oxide copolymer (average degree of polymerization of propylene oxide/ethylene oxide: about 60/180, PLURONIC F-127 manufactured by Asahi Denka Co., Ltd.) was dissolved in 30 mL of dry chloroform, 0.13 g of hexamethylene diisocyanate is added in the presence of phosphorus pentoxide, and allowed to react for six hours under boiling point reflux. The solvent was evaporated under reduced pressure, the residue was dissolved in distilled water, and ultrafiltration was carried out using an ultrafiltration membrane having a molecular weight cutoff of 500,000, thereby fractionating a high molecular weight polymer and a low molecular weight polymer. The aqueous solution thus obtained was frozen, thereby obtaining an F-127 high molecular weight polymer and an F-127 low molecular weight polymer.

1 g of the F-127 high molecular weight polymer obtained as described above (hydrogel-forming polymer of the present invention, "hydrogel-forming polymer"-1) was dissolved in 9 g of distilled water under cooling with ice, thereby obtaining a 10 wt% aqueous solution. The storage elastic modulus of the aqueous solution was 4 Pa at 10°C, 1890 Pa at 25°C, and 6660 Pa at 37°C as measured using a stress controlled rheometer (AR500, manufactured by TA Instruments) at an application frequency of 1 Hz. The temperature-dependent change in the storage elastic modulus was reversibly and repeatedly observed. Meanwhile, the solution of the F-127 low molecular weight polymer in distilled water at a concentration of 10 wt% at a freezing point did not cause gelation even when heated to 60°C or higher.

### Preparation Example 2

To 1 mol of trimethylolpropane, 160 mol of ethylene oxide was added by cationic polymerization, thereby obtaining polyethylene oxide triol having an average molecular weight of about 7000.

100 g of the polyethylene oxide triol obtained as described above was dissolved in 1000 mL of distilled water, 12 g of filtrated potassium manganate was gradually added at room temperature, and allowed to cause oxidation reaction for about one hour. The solid was removed by filtration, the product was extracted with chloroform, and the solvent (chloroform) was evaporated under reduced pressure, thereby obtaining 90 g of polyethylene oxide tricarboxylate.

10 g of the polyethylene oxide tricarboxylate obtained as described above and 10 g of polypropylene oxide diamine (average degree of polymerization of propylene oxide: about 65, Jefferson Chemical Company in the US, trade name: JEFFAMINE D-4000, cloud point: about 9°C) were dissolved in 1000 mL of carbon tetrachloride, 1.2 g of dicyclohexyl carbodiimide was added, and then allowed to react for 6 hours under boiling point reflux. The reaction liquid was cooled, the solid was removed by filtration, the solvent (carbon tetrachloride) was evaporated under reduced pressure, the residue was dried in vacuo, thereby obtaining the hydrogel-forming polymer ("hydrogel-forming polymer"-2) of the present invention to which multiple polypropylene oxide and polyethylene oxide are bonded. 1 g of the polymer was dissolved in 19 g of distilled water under cooling with ice, thereby obtaining a 5 wt% aqueous solution. The storage elastic modulus of the aqueous solution was 1 Pa at 10°C, 550 Pa at 25°C, and 3360 Pa at 37°C as measured using a stress controlled rheometer (AR500, manufactured by TA Instruments) at an application frequency of 1 Hz. The temperature-dependent change in the storage elastic modulus was reversibly and repeatedly observed.

### Preparation Example 3

96 g of N-isopropyl acrylamide (manufactured by Eastman Kodak Company), 17 g of N -acryloxysuccinimide (manufactured by Kokusan Chemical Co., Ltd.), and 7 g of n-butyl methacrylate (manufactured by Kanto Chemical Co., Inc.) were dissolved in 4000 mL of chloroform, and after purging with nitrogen, 1.5 g of N,N≡-azobisisobutyronitrile was added, and allowed to polymerize at 60°C for 6 hours. The reaction liquid was concentrated, and reprecipitated in diethyl ether. The solid was collected by filtration, dried in vacuo, thereby obtaining 78 g of poly(N-isopropyl acrylamide -co-N-acryloxysuccinimide-co-n-butyl methacrylate).

To the poly(N-isopropyl acrylamide-co-N-acryloxysuccinimide-co-n-butyl methacrylate) obtained as described above, excessive isopropylamine was added, thereby obtaining poly(N-isopropyl acrylamide-co-n-butyl methacrylate). The cloud point of the aqueous solution of the poly(N-isopropyl acrylamide-co-n-butyl methacrylate) was 19°C.

10 g of the poly(N-isopropyl acrylamide-co-N-acryloxysuccinimide-co-n-butyl methacrylate) and 5 g of polyethylene oxide having amino groups at both ends (molecular weight: 6,000, manufactured by Kawaken Fine Chemicals Co., Ltd.) were dissolved in 1000 mL of chloroform, and allowed to react at 50°C for 3 hours. After cooling to room temperature, 1 g of isopropylamine was added, the mixture was allowed to stand for 1 hour, then the reaction liquid was concentrated, and the residue was precipitated in diethyl ether. The solid was collected by filtration, and dried in vacuo, thereby obtaining the hydrogel-forming polymer ("hydrogel-forming polymer"-3) of the present invention to which multiple molecules of poly(N-isopropyl acrylamide-co-n-butyl methacrylate) and polyethylene oxide were bonded. 1 g of the polymer was dissolved in 9 g of distilled water under cooling with ice, thereby obtaining a 10 wt% aqueous solution. The storage elastic modulus of the aqueous solution was 1 Pa or less at 10°C, 30 Pa at 25°C, and 250 Pa at 37°C as measured using a stress controlled rheometer (AR500, manufactured by TA Instruments) at an application frequency of 1 Hz. The temperature-dependent change in the storage elastic modulus was reversibly and repeatedly observed.

### Preparation Example 4

### (Sterilization method)

2.0 g of the above-described hydrogel-forming polymer ("hydrogel-forming polymer"-3) of the present invention was placed in an EOG (ethylene oxide gas) sterilization bag (trade name: Hybrid Sterilized Bag, manufactured by Hogy Medical Co., Ltd.), the bag was filled with EOG with an EOG sterilization apparatus (Easy-Pack, manufactured by As One Corporation), and the bag was allowed to stand overnight at room temperature. Furthermore, the bag was allowed to stand half-day at 40°C, and then EOG was extracted from bag, and aeration was carried out. The bag was placed in a vacuum desiccator (40°C), and allowed to stand half-day under occasional aeration, thereby achieving sterilization.

As a result of the sterilization operation, it was confirmed anew that the storage elastic modulus of the polymer aqueous solution did not change.

### Preparation Example 5

71.0 g of N-isopropyl acrylamide and 4.4 g of n-butyl methacrylate were dissolved in 1117 g of ethanol. To the solution, an aqueous solution prepared by dissolving 22.6 g of polyethylene glycol dimethacrylate (PDE6000, manufactured by NOF Corporation) in 773 g of water was added, and humidified to 70°C in a nitrogen gas stream. In a nitrogen gas stream, 0.8 mL of N,N,N=,N≡-tetramethylethylenediamine (TEMED) and 8 mL of 10% ammonium persulfate (APS) aqueous solution were added while the temperature was kept at 70°C, and allowed to react for 30 minutes under stirring. Furthermore, 0.8 mL of TEMED and 8 mL of 10% APS aqueous solution were added four times at intervals of 30 minutes, thereby completing polymerization reaction. After cooling the reaction liquid to 10°C or lower, 5 L of cooled distilled water at 10°C was added for dilution, and the liquid was concentrated to 2 L at 10°C using an ultrafiltration membrane having molecular weight cut off of 100,000.

To the concentrate, 4 L of cooled distilled water was added for dilution, and the ultrafiltration and concentration operation was carried out again. The above-described operations of dilution and ultrafiltration/concentration were further repeated five times, thereby removing those having a molecular weight of 100,000 or less. The portion which had not been filtered through by the ultrafiltration (the portion that remained in the ultrafiltration membrane) was collected and freeze-dried, thereby obtaining 72 g of the hydrogel-forming polymer ("hydrogel-forming polymer"-5) of the present invention having a molecular weight of 100,000 or more.

1 g of the thus obtained hydrogel-forming polymer ("hydrogel-forming polymer"-5) of the present invention was dissolved in 9 g of distilled water under cooling with ice, thereby obtaining a 10 wt% aqueous solution. The storage elastic modulus of the aqueous solution was 1 Pa or less at 10°C, 80 Pa at 25°C, and 460 Pa at 37°C as measured using a stress controlled rheometer (AR500, manufactured by TA Instruments) at an application frequency of 1 Hz. The temperature-dependent change in the storage elastic modulus was reversibly and repeatedly observed.

### Preparation Example 6

42.0 g of N-isopropyl acrylamide and 4.0 g of n-butyl methacrylate were dissolved in 592 g of ethanol. To the solution, an aqueous solution prepared by dissolving 11.5 g of polyethylene glycol dimethacrylate (PDE6000, manufactured by NOF Corporation) in 65.1 g of water was added, and warmed to 70°C in a nitrogen gas stream. In a nitrogen gas stream, 0.4 mL of N,N,N=,N=-tetramethylethylenediamine (TENED) and 4 mL of 10% ammonium persulfate (APS) aqueous solution were added while the temperature was kept at 70°C, and allowed to react at intervals of 30 minutes. Furthermore, 0.4 mL of TENED and 4 mL of 10% APS aqueous solution were added four times at intervals of 30 minutes, thereby completing polymerization reaction. After cooling the reaction liquid to 59°C or lower, 5 L of cooled distilled water at 5°C was added for dilution, and the liquid was concentrated to 2 L at 5°C using an ultrafiltration membrane having molecular weight cut off of 100,000.

To the concentrate, 4 L of cooled distilled water was added for dilution, and the ultrafiltration and concentration operation was carried out again. The above-described operations of dilution and ultrafiltration/concentration were further repeated five times, thereby removing those having a molecular weight of 100,000 or less. The portion which had not been filtered through by the ultrafiltration (the portion that remained in the ultrafiltration membrane) was collected and freeze-dried, thereby obtaining 40 g of the hydrogel-forming polymer ("hydrogel-forming polymer"-6) of the present invention having a molecular weight of 100,000 or more.

1 g of the thus obtained hydrogel-forming polymer ("hydrogel-forming polymer"-6) of the present invention was dissolved in 9 g of distilled water under cooling with ice, thereby obtaining a 10 wt% aqueous solution. The storage elastic modulus of the aqueous solution was 43 Pa at 10°C, 680 Pa at 25°C, and 1310 Pa at 37°C as measured using a stress controlled rheometer (AR500, manufactured by TA Instruments) at an application frequency of 1 Hz. The temperature-dependent change in the storage elastic modulus was reversibly and repeatedly observed.

### Preparation Example 7

45.5 g of N-isopropyl acrylamide and 0.56 g of n-butyl methacrylate were dissolved in 592 g of ethanol. To the solution, an aqueous solution prepared by dissolving 11.5 g of polyethylene glycol dimethacrylate (PDE6000, manufactured by NOF Corporation) in 65.1 g of water was added, and warmed to 70°C in a nitrogen gas stream. In a nitrogen gas stream, 0.4 mL of N,N,N=,N=-tetramethylethylenediamine (TENED) and 4 mL of 10% ammonium persulfate (APS) aqueous solution were added while the temperature was kept at 70°C, and allowed to react for 30 minutes under stirring. Furthermore, 4.0 mL of TENED and 4 mL of 10% APS aqueous solution were added four times at intervals of 30 minutes, thereby completing polymerization reaction. After cooling the reaction liquid to 10°C or lower, 5 L of cooled distilled water at 10°C was added for dilution, and the liquid was concentrated to 2 L at 10°C using an ultrafiltration membrane having molecular weight cut off of 100,000.

To the concentrate, 4 L of cooled distilled water was added for dilution, and the ultrafiltration and concentration operation was carried out again. The above-described operations of dilution and ultrafiltration/concentration were further repeated five times, thereby removing those having a molecular weight of 100,000 or less. The portion which had not been filtered through by the ultrafiltration (the portion that remained in the ultrafiltration membrane) was collected and freeze-dried, thereby obtaining 22 g of the hydrogel-forming polymer ("hydrogel-forming polymer"-7) of the present invention having a molecular weight of 100,000 or more.

1 g of the thus obtained hydrogel-forming polymer ("hydrogel-forming polymer"-7) was dissolved in 9 g of distilled water under cooling with ice, thereby obtaining a 10 wt% aqueous solution. The storage elastic modulus of the aqueous solution was 1 Pa or less at 10°C, 1 Pa or less at 25°C, and 90 Pa at 37°C as measured using a stress controlled rheometer (AR500, manufactured by TA Instruments) at an application frequency of 1 Hz. The temperature-dependent change in the storage elastic modulus was reversibly and repeatedly observed.

### Example 1

The freeze-dried hydrogel-forming polymer-6 obtained in Preparation Example 6 was subjected to EOG sterilization in the same manner as in Preparation Example 4. The hydrogel-forming polymer-6 after EOG sterilization was dissolved in a phosphate buffer solution under cooling with ice at a concentration of 10 wt%. The oral mucosal tissues (2 cm × 1 cm) of a male patient with urethral stricture were collected, the serum (an equivalent amount of the cells) of the patient himself was added to collagenasetreated cells, and dispersed in a phosphate buffer solution of the hydrogel-forming polymer-6 under cooling with ice. The cell dispersion was heated to 37°C and gelated, and cultured for 10 days, thereby increasing the number of cells to about 10 times. The urethral stricture site of the patient was incised by a transurethral endoscopic incision procedure, a urethra catheter was inserted, and then the urethral stricture treatment agent of the present invention cooled with ice after the above-described cell cultivation was injected between the periphery of the urethra catheter and the incised site of the inner surface of urethra. The urethral stricture treatment agent of the present invention was warmed by body temperature and instantly gelated, and held so as to cover the whole incised site of the inner surface of urethra. The urethra catheter was extracted three weeks after surgery. The site with incised stricture was covered by mucosal epithelial cells without causing scarring, and good flow of urine was ensured. The same treatment was carried out on 10 patients, and no restenosis was observed in any of the patients.

### Example 2

The same treatment as in Example 1 was carried out on 10 patients except that the oral mucosal cells and the serum of a male patient with urethral stricture were not added. As a result, restenosis was not observed in any of the patients, but scarring of the site with incised stricture was observed in two patients.

### Example 3

The same treatment as in Example 2 was carried out on 10 patients except that the freeze-dried hydrogel-forming polymer-5 obtained in Preparation Example 5 was used in place of the freeze-dried hydrogel-forming polymer-6 obtained in Preparation Example 6. As a result, restenosis was observed in two patients.

### Comparative Example 1

The same treatment as in Example 1 was carried out on 10 patients except that the freeze-dried hydrogel-forming polymer-7 obtained in Preparation Example 7 was used in place of the freeze-dried hydrogel-forming polymer-6 obtained in Preparation Example 6. As a result, scarring of the site with incised stricture was observed in all the patients. The reason for this seems to be that the storage elastic modulus of the hydrogel-forming polymer-7 was as low as less than 100 Pa at 37°C, so that the agent did not function as the urethral stricture treatment agent of the present invention.

### Comparative Example 2

In Example 1, the freeze-dried hydrogel-forming polymer-6 after EOG sterilization was dissolved in a phosphate buffer solution at a concentration of 11 wt% under cooling with ice. The storage elastic modulus of the aqueous solution was 75 Pa at 10°C as measured using a stress controlled rheometer (AR500, manufactured by TA Instruments) at an application frequency of 1 Hz. The solution had a low flowability even under cooling with ice, and could not be injected into the urethral stricture site through a catheter, so did not function as the urethral stricture treatment agent of the present invention.

## Claims

1. A urethral stricture treatment agent comprising at least a hydrogel-forming polymer, and having a storage elastic modulus of 50 Pa or less at 10°C and a storage elastic modulus of 100 Pa or more at 37°C.

2. The urethral stricture treatment agent according to claim 1, comprising animal cells.

3. The urethral stricture treatment agent according to claim 2, wherein the animal cells are oral mucosal cells of a patient.

4. A urethral stricture treatment method at least comprising:
injecting the urethral stricture treatment agent according to claim 1 cooled to 10°C or lower into an inner surface of urethra which has been incised with a transurethral endoscopic procedure; and
holding the urethral stricture treatment agent in the inner surface of urethra at a temperature not lower than a room temperature.
